(19) Europäisches Patentamt
European Patent Office
Office européen des brevets

(11) Publication number: **0 031 603 A1**

# (12) EUROPEAN PATENT APPLICATION

(21) Application number: 80201115.5

(22) Date of filing: 25.11.80

(51) Int. Cl.³: A 61 K 31/245

(30) Priority: 31.12.79 US 109015

(43) Date of publication of application: 08.07.81 Bulletin 81/27

(84) Designated Contracting States: BE CH DE FR GB IT LI NL SE

(71) Applicant: American Cyanamid Company, 1937 West Main Street, Stamford Connecticut 06904 (US)

(72) Inventor: Krueger, James Elwood, 6 Lucille Boulevard, New City New York 10956 (US)
Inventor: Bernardo, Peter D., RFD 3, Mt. Holly Road, Katonah New York 10536 (US)
Inventor: Welch, Simon Alan, 7 Wesley Road, Congers New York 10920 (US)

(74) Representative: Wächtershäuser, Günter, Dr., Gewürzmühlstrasse 5, D-8000 München 22 (DE)

## (54) Pharmaceutical composition of matter.

(57) A composition for improved absorption of drugs which are poorly water-soluble and poorly absorbed. Particularly this invention relates to a composition of matter in pharmaceutical dosage form comprising 4-(monoalkylamino)benzoic acid and derivatives and non-ionic surfactants, providing enhanced blood levels of 4-(monoalkylamino)benzoic acid and derivatives for the treatment of atherosclerosis hypolipidemia. A method of increasing drug absorption (particularly 4-(monoalkylamino)benzoic acid and derivatives) by administration of such drug in concert with greater than about 10% surfactant is described.

EP 0 031 603 A1

TITLE: PHARMACEUTICAL COMPOSITION OF MATTER

## FIELD OF THE INVENTION

This invention relates to a composition for improved absorption of drugs which are poorly water-soluble and poorly absorbed. Particularly, this invention relates to a composition of matter in pharmaceutical dosage form comprising 4-(monoalkylamino)-benzoic acid and derivatives and non-ionic surfactants, providing enhanced blood levels of 4-(monoalkylamino)benzoic acid and derivatives for the treatment of atherosclerosis hypolipidemia. A method of increasing drug absorption (particularly 4-(monoalkylamino)benzoic acid and derivatives) by administration of such drug in concert with greater than about 10% surfactant is described.

## BACKGROUND OF THE INVENTION

4-(monoalkylamino)benzoic acid and derivatives has been found to be an effective pharmaceutical agent in the treatment of atherosclerosis and hypolipidemia. The general range of effective blood levels of this drug is generally from about 1 microgram per ml. to about 10 milligrams per ml. However, a relatively low rate of absorption of polyalkylaminobenzoic acid across the gut wall has required relatively large oral dosages to be administered to effect an effective final concentration. Doses of 100 to 300 mg./kg. (body weight) gave less than 1 microgram per milligram serum level. A number of substances have been investigated and found to enhance transport of polyalkylaminobenzoic acid across the gut wall. Among these are coprecipitates of polyalkylaminobenzoic acid and derivatives and polyhydroxylic compounds such as lactose, and polyethylene glycol, U.S Application Serial No. ___,___ [Attorney's Docket No. 27,804], filed on even date herewith. Another is a coprecipitate with polyvinylpyrrolidine which is the subject of Application U.S. Serial No. _______ [Attorney's docket No. 27,176] filed on even date herewith.

PRIOR ART

1) U.S. Patent No. 3,868,416 (ACCO)
2) Consecutive ACCO Cases 26,896-26,909
3) U.S. Patent No. 3,673,163
4) P. Molyneux and H. Frank, J.A.C.S., 83, 3169 (1961)
5) T. Higushi, *et al.*, J. Am. Pharm. Assoc. Sci. Ed., 43, 393 (1954) and 398 (1954)
6) M. Mayersohn, *et al.*, J. Pharm. Sci., 55, 1323 (1966)
7) A. P. Simonelli, *et al.*, J. Pharm. Sci., 58, 538 (1959)
8) J. P. Davignon, Bull. Parent. Drug Assoc., 28, 83 (1969)

DESCRIPTION OF THE INVENTION

This invention is particularly concerned with a composition of matter comprising a compound of the formula:

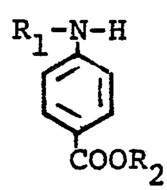

wherein $R_1$ is an unbranched or branched alkyl group $C_nH_{2n+1}$ wherein n is 8 to 19 and $R_2$ is hydrogen, lower alkyl, benzyl, dilower alkylaminoethyl, or lower alkoxyethyl together with the pharmaceutically acceptable salts thereof (hereafter polyalkylaminobenzoic acid) and non-ionic surfactants.

The above described compounds, their use as hypolipidemic agents and conventional pharmaceutical dosage forms for these products are fully disclosed in U.S. Patent

No. 3,868,416.

It has now been discovered that a composition of matter comprising of one of the aforementioned compounds and non-ionic surfactants provides a product which, when administered orally to warm-bloodied animals, provides for substantially enhanced blood levels of the therapeutic component when compared with the oral administration of one of the aforementioned compounds alone.

A particularly surprising aspect of this invention is the discovery that substances that may be absorbed only slowly from the intestinal tract find enhanced absorption when in combination with surfactant at much higher levels of surfactant than had been hitherto thought useful. These levels of surfactant must be about 10% (W/W). Further, the surfactant need not dissolve the drug, a mixture or suspension as in the case of polyalkylaminobenzoic acid, being suitable.

Drugs which are poorly water-soluble, and poorly absorbed, find enhanced absorption in the composition of this invention. The preferred drug:surfactant ratio range is from about 1:33 to 1:1 (i.e. about 3% drug to about 50% drug). Most preferred is a composition of about 3 to about 27% drug.

The percentages of surfactant previously used are described in Micellization, Solubilization, and Microemulsions Vol. 1 K.L. Mittal (plenum Press, 1977) particularly p. 66 et seq.

Clearly the enhancement of absorption is dependent on how poorly the drug is absorbed without surfactant. Further, in keeping with a suggestion, increased solubilization into intestinal micelles being a possible method of increasing absorption, an increased lipophilic nature of the drug will cause an increase in the enhancement of absorption.

This effect is true for all drugs that are poorly absorbed from the intestinal tract and particularly those which are lipophilic.

Some of the drugs suitable for particular enhancement of absorption are the poorly absorbed, poorly water-soluble members of the following classes:

CNS agents* such as anticonvulsants (e.g. phenacemide, phenantoin) particularly diphenylhydantoin, autonomic drugs such as skeletal muscle relaxants (e.g. chlorphenesin carbamate, mephenesin carbamate) particularly methocarbamol; hormones such as steroids and corticosteroids (e.g. fluprednisdone, meprednisone), particularly prednisone; anti-infective agents such as antifungal antibiotics (e.g. emetine, glycobiarsol) particularly griseofulvin; cardiovascular agents such as hypocholesteremics (e.g. clofibrate, dextrothyroxine) particularly probucol.

The preferred non-ionic surfactants are generally the polysorbates, particularly polysorbate 60, polysorbate 65, polysorbate 80, and polysorbate 85 with polysorbate 80 being most preferred. Polysorbate 80 is (Z)-sorbitan mono-9-octadecenoate, polyoxyethylene derivative.

The preferred compositions are in the form of a mixture or suspension of the drug (particles of 50 microns or less, (average diameter)) with a surfactant. The smallest average particle sizes are most preferred. The p-hexadecylaminobenzoic acid sodium salt is the preferred form of the active substance.

This composition is prepared as a 5% to 50% aqueous suspension of for example p-hexadecylaminobenzoic acid sodium salt, initially containing 2% to 50% polysorbate 80. Heat may be used to affect suspension and the water may or may not be removed from the suspension after formation but the final surfactant concentration is at least about 10%.

Non-aqueous suspensions and oil-in-water emulsions are also prepared. Suitable oils are sesame oil,

* The categories cited are those of the Hospital Formulary of the American Society of Hospital Pharmacies.

olive oil, mineral oil, rape seed oil and other pharmaceutically acceptable oils.

The p-hexadecylaminobenzoic acid compositions of this invention are administered to warm-blooded animals in amounts ranging from about 5 mg. to about 200 mg. (based on the active component, p-hexadecylaminobenzoic acid sodium salt content) per kilogram of body weight per day. A preferred dosage regimen for optimum results would be from about 5 mg. to about 50 mg. (of active component) per kilogram of body weight per day and such dosage units are employed that a total of from about 350 mg. to about 3.5 g. of the active component for a subject of about 70 kg. of body weight are administered in a 24 hour period. This dosage regimen may be adjusted to provide the optimum therapeutic response. For example, several divided doses may be administered daily or the dose may be proportionally reduced as indicated by the exigencies of the situation. A decided practical advantage of this invention is that the composition may be administered orally. Dosage forms wherein the therapeutic compound comprises up to 90% of the dosage form by weight may be used, surfactant of at least about 10% of the dosage weight is usually required though dosage of about 3 to 50% therapeutic compound is preferred and about 3% to 29% therapeutic compounds are most preferred.

This composition may be administered orally, for example, with an inert diluent or an assimilable edible carrier, or it may be enclosed in soft shell gelatin capsules, or it may be incorporated directly with the food of the diet.

The bioavailability of the compositions of this invention was compared with that of p-hexadecylaminobenzoic acid sodium salt by the following procedures.

Normal and $^{14}C$-labeled p-hexadecylaminobenzoic acid sodium salt were formed in compositions with polysorbate 80 at varying concentrations, according to procedures given in the following examples and filled in soft

shell gelatin capsules or filled as single doses in 5 ml. Glaspak$^R$ syringes.

Control capsules were prepared by mixing and spatulating for 5 minutes 1.320 g. of normal or $^{14}C$-labeled p-hexadecylaminobenzoic acid sodium salt, 1.776 g. of lactose monohydrate and 64.8 mg. of modified starch. About 0.5 ml. of water was added dropwise with mixing to form granules, which were then dried at 50°C. for 18 hours. The granules were passed through a No. 17 sieve, 32.4 mg. of sodium lauryl sulfate and 32.4 mg. of magnesium stearate were passed through the sieve and mixed with the granules by spatulation for 3 minutes and the resultant mixture was then filled in hard shell gelatin capsules.

The particle sizes of active material in all mixtures or capsules were qualitatively similar.

The capsule formulations were administered orally to dogs by hand insertion and the other compositions were administered by gavage. The dogs were purebred male beagles from Marshall Research Animals Inc., North Rose, New York. All dose administrations were followed by a 30 ml. water wash.

The capsule formulations were administered orally to monkeys by use of a balling gun and other compositions by gavage. The monkeys were male and female Macaca Fascicularis monkeys from Primate Imports, Port Washington, New York. All dose administrations were followed by a 2 ml. wash of grape juice.

All animals had free access to water at all times. They were fed in the morning of the days preceding and following the day of drug administration. Half of the monkeys in the comparative fed vs. fasted study were fed their normal diet one hour prior to drug administration. For those studies in which blood was collected, the animals were not fed again until after the 23 or 24 hour blood samples were collected.

Each dog was fed 300 g. of Respond 2000$^{TM}$ dog food (Agway Country Foods, Waverly, New York) daily.

On the days of feeding, the monkeys were provided Purina Monkey Chow (Ralston Purina Co., St. Louis, Missouri) in the morning and given bread and fruit in the afternoon.

The dogs were housed in individual cages designed to collect the voided urine separately from the feces. The urine was collected at room temperature in individual two liter polyethylene containers. The collection pans were washed with 50-100 ml. of water after each urine collection and each rinse was added to the respective daily collection of urine.

Blood samples were obtained from the monkeys from their femoral artery and injected immediately into 10 ml. Corvac$^R$ tubes and allowed to clot. The tubes were centrifuged and the sera were pipetted directly into both counting vials and acid-washed conical tubes. The samples for fluorometric analysis were frozen until analyzed.

The radioactivity measurements were made as follows: All samples were counted in 10 ml. 3a70B phosphor (Research Products International Corp.) using a Beckman LS9000 Liquid Scintillation Spectrophotometer. Two drops of saturated aqueous ascorbic acid solution were added to each sample to minimize chemiluminescence. Conversion to absolute radioactivity was performed by use of the internal computer synthesized channels ratio calibration curve based on sealed quenched carbon-14 standards.

Daily urine collection samples of less than one liter were diluted to one liter with water and mixed. Urine samples of one ml. each were counted in either triplicate or quadruplicate for each time period. Single and duplicate 0.5 ml. serum samples were analyzed. One-half ml. of water was added to each serum-containing vial to prevent gel separation of the final counting mixture.

The serum samples were assayed by a fluorometric method as follows: Aliquot volumes of from 0.1 to 0.5 ml. were analyzed. For samples of less than 0.5 ml., water was

added to bring the volume to 0.5 ml. The samples were transferred to acid washed 15 ml. glass stoppered centrifuge tubes and 0.4 ml. of water and 0.5 ml. of freshly prepared 4% potassium hydroxide in 95% ethanol were added. The contents were mixed on a vortex mixer, stoppered and heated for 75 minutes at 95°C., then cooled to room temperature. A 3.5 ml. portion of water:acetic acid (4:3) was added and the contents were mixed. Eight ml. of hexane containing 3% isoamyl alcohol was added, the contents were shaken vigorously and then centrifuged. A 3 ml. portion of the organic layer was transferred to a cuvette and the fluorescence was measured on a Perkin-Elmer Model 1000 Fluorescence Spectrophotometer equipped with a constant voltage power supply; excitation:300 nm. Oriel interference filter; emission:339 nm. Perkin-Elmer interference filter, mirrored side facing sample compartment, narrow slit width, scale expansion knob turned fully counter-clockwise. The results were compared to a standard curve plotted from results obtained in the same manner using samples of $^{14}$C-labeled 4-(hexadecylamino)benzoic-carboxy-$^{14}$C-acid, 2,3-dihydroxypropyl ester.

The results of these tests appear in the following tables, I, II, III, IV and V.

TABLE I

Summary of the Urinary Excretion Data from Bioavailability Studies in Fasted Dogs Expressed in Percent of Administered Doses

| Formulation | Dog No. | Weight (kg.) | Dose* (mg./kg.) | Urinary Excretion (% of Dose*) Time in Hours 0-24 | 24-48 | 48-72 | 72-96 | 0-96 |
|---|---|---|---|---|---|---|---|---|
| Control Capsule | 20753 | 7.4 | 14.7 | 5.1 | 2.0 | 2.1 | 0.7 | 9.9 |
| | 20767 | 9.8 | 11.1 | 4.9 | 2.3 | 1.1 | 0.4 | 8.7 |
| | 20779 | 8.3 | 13.1 | 8.2 | 5.2 | 5.6 | 2.4 | 21.4 |
| | 20797 | 9.1 | 11.9 | 6.1 | 3.3 | 1.5 | 0.9 | 11.8 |
| Polysorbate 80:Drug* (33:1) Suspension | 26251 | 7.80 | 11.5 | 10.5 | 14.7 | 3.9 | 3.0 | 32.1 |
| | 26253 | 10.70 | 7.4 | 5.7 | 13.6 | 1.5 | 2.3 | 23.1 |
| | 26281 | 8.00 | 11.0 | 0.4 | 12.8 | 2.5 | 1.7 | 17.4 |
| Polysorbate 80:Drug* (1:1) Capsule | 26259 | 7.90 | 14.1 | 3.3 | 2.7 | 0.9 | 0.6 | 7.5 |
| | 26264 | 10.00 | 11.1 | 4.2 | 15.5 | 4.5 | 3.5 | 27.7 |
| | 26285 | 8.70 | 12.8 | 0.0 | 5.8 | 1.6 | 0.9 | 8.3 |
| Polysorbate 80:Drug*:Lecithin (1:1:0.1) Capsule | 26250 | 8.10 | 14.8 | 1.6 | 7.9 | 1.9 | 1.0 | 12.4 |
| | 26260 | 9.60 | 12.5 | 0.4 | 5.1 | 2.9 | 1.0 | 9.4 |
| | 26286 | 8.80 | 13.6 | 2.6 | 5.7 | 1.2 | 0.7 | 10.2 |

* $^{14}C$-Labeled p-hexadecylaminobenzoic acid sodium salt

TABLE II

Summary of the Mean 0-96 Hour Urinary Excretion Data from Bioavailability Studies in Fasted Dogs

| Formulation | No. of Dogs | Mean Dose* (mg./kg.) | Mean Percent of Dose in 0-96 Hour Urine |
|---|---|---|---|
| Control Capsule | 4 | 12.7 | 13.0 |
| Polysorbate 80:Drug* (33:1) | 3 | 10.0 | 24.2 |
| Polysorbate 80:Drug* (1:1) | 3 | 12.7 | 14.5 |
| Polysorbate 80:Drug*:Lecithin (1:1:0.1) | 3 | 13.6 | 10.7 |

* $^{14}C$-Labeled p-hexadecylaminobenzoic acid sodium salt

TABLE III

Summary of the Serum Drug Concentrations Determined by the Radiometric and Fluorometric Assays from Bioavailability Studies in Fasted Dogs

| Formulation | Animal No. | Weight kg. | Dose mg./kg. | mcg. of Drug* Equivalents/ml. of Serum (Fluorometric Results in Parenthesis) Time in Hours 2 | 4 | 6 | 8 | 10 | 12 | 24 | 48 | 0-48 Hr. AUC** |
|---|---|---|---|---|---|---|---|---|---|---|---|---|
| Control Capsule | | | | 1.62 (1.50) | 2.14 (1.87) | 1.72 (1.49) | 1.27 (1.12) | 1.05 (0.72) | 0.84 (0.58) | 0.54 (0.44) | 0.52 (0.40) | 37.4 (30.2) |
| Polysorbate 80:-Drug* (33:1) Suspension | 26251 | 7.80 | 11.5 | 6.5 (5.8) | 5.6 (4.6) | 4.0 (3.2) | 3.5 (2.8) | 3.1 (2.5) | 2.7 (2.1) | 1.8 (1.4) | 1.1 (0.8) | 109.9 (87.3) |
| | 26253 | 10.70 | 7.4 | 3.0 (2.8) | 3.1 (2.6) | 2.5 (2.0) | 2.2 (1.7) | 1.9 (1.6) | 1.6 (1.3) | 1.1 (0.8) | 0.8 (0.4) | 66.0 (49.7) |
| | 26281 | 8.00 | 11.0 | 4.9 (4.6) | 5.5 (4.6) | 4.0 (3.3) | 3.3 (2.7) | 2.8 (2.2) | 2.4 (1.9) | 1.1 (0.9) | 0.7 (0.5) | 86.0 (70.3) |
| Polysorbate 80:-Drug* (1:1) Capsule | 26259 | 7.90 | 14.1 | 0.9 (1.0) | 2.7 (2.3) | 2.0 (1.6) | 1.6 (1.2) | 1.4 (1.1) | 1.2 (0.8) | 0.8 (0.6) | 0.9 (0.6) | 50.8 (38.0) |
| | 26264 | 10.00 | 11.1 | 1.1 (1.0) | 2.4 (1.9) | 1.9 (1.5) | 1.4 (1.0) | 2.0 (1.6) | 3.1 (2.5) | 1.7 (1.3) | 1.0 (0.6) | 81.9 (62.1) |
| | 26285 | 8.70 | 12.8 | 0.8 (0.8) | 1.8 (1.7) | 1.7 (1.5) | 1.7 (1.4) | 1.5 (1.2) | 1.3 (1.1) | 0.6 (0.5) | 0.4 (0.3) | 39.7 (33.5) |
| Polysorbate 80:-Drug*:Lecithin (1:1:0.1) Capsule | 26250 | 8.10 | 14.8 | 1.2 (1.1) | 2.3 (2.0) | 2.2 (1.7) | 1.9 (1.4) | 1.5 (1.1) | 1.2 (0.9) | 0.6 (0.4) | 0.5 (0.3) | 43.4 (31.7) |
| | 26260 | 9.60 | 12.5 | 1.4 (1.3) | 1.7 (2.1) | 1.6 (1.2) | 1.5 (1.1) | 1.2 (1.0) | 1.1 (0.8) | 0.7 (0.6) | 0.7 (0.4) | 43.5 (34.6) |
| | 26286 | 8.80 | 13.6 | 1.7 (1.4) | 3.5 (3.1) | 2.5 (1.9) | 2.0 (1.6) | 1.6 (1.3) | 1.4 (1.0) | 0.6 (0.4) | 0.4 (0.2) | 48.0 (35.2) |

* $^{14}C$-Labeled p-hexadecylaminobenzoic acid sodium salt
**AUC = Area under the serum drug concentration - time curve (mcg.-hr./ml.)

TABLE IV

Summary of Serum Drug Concentrations Determined by Radiometric and Fluorometric Assays from Bioavailability Studies in Fasted Monkeys

| Formulation | Animal No. | Weight kg. | Dose mg./kg. | mcg. of Drug* Equivalents/ml. of Serum (Fluorometric Results in Parenthesis) | | | | | | | |
|---|---|---|---|---|---|---|---|---|---|---|---|
| | | | | Time in Hours | | | | | | | 0-48 Hr. AUC** |
| | | | | 2 | 4 | 6 | 10 | 14 | 23 | 48 | |
| Control Capsule | 13631 | 5.35 | 20.8 | 4.8 (3.7) | 2.2 (1.6) | 1.2 (0.9) | 0.7 (0.5) | 0.4 (0.2) | 0.3 (0.1) | 0.1 (0.0) | 29.6 (18.6) |
| | 23130 | 3.25 | 34.3 | 3.6 (3.3) | 1.4 (1.1) | 0.7 (0.5) | 0.4 (0.2) | 0.3 (0.1) | 0.3 (0.1) | 0.1 (0.0) | 22.2 (13.5) |
| | 18723 | 2.45 | 45.5 | 9.4 (3.7) | 11.1 (9.2) | 4.3 (3.5) | 2.3 (1.8) | 1.4 (1.0) | 0.9 (0.4) | 0.2 (0.1) | 90.6 (58.9) |
| Polysorbate 80:- Drug* (33:1) Suspension | 23119 | 4.35 | 23.3 | 16.6 (13.3) | 67.6 (61.9) | 41.8 (42.7) | 12.4 (9.6) | 6.6 (5.5) | 2.9 (1.7) | 0.8 (0.4) | 447.4 (387.6) |
| | 20110 | 4.20 | 24.1 | 25.7 (20.3) | 52.7 (44.3) | 43.1 (36.7) | 13.8 (11.9) | 7.3 (5.4) | 2.7 (1.7) | 0.8 (0.5) | 446.4 (359.3) |
| | 20132 | 3.55 | 28.5 | 20.9 (19.7) | 45.4 (42.9) | 44.4 (42.4) | 22.2 (20.0) | 8.8 (8.0) | 3.4 (2.4) | 0.9 (0.9) | 483.0 (438.1) |
| Polysorbate 80:- Drug* (10:1) Capsule | 23181 | 2.30 | 46.4 | (35.5) | (73.8) | (30.6) | (10.8) | (4.2) | (1.6) | (0.5) | (416.2) |
| | 23176 | 2.60 | 41.1 | (46.5) | (18.9) | (8.3) | (4.2) | (2.1) | (0.8) | (0.4) | (203.8) |
| | 23140 | 3.80 | 28.1 | (43.0) | (16.3) | (7.0) | (2.9) | (1.4) | (0.9) | (0.2) | (178.8) |
| Polysorbate 80:- Drug* (5:1) Capsule | 23184 | 2.55 | 42.4 | (30.8) | (27.0) | (13.8) | (4.4) | (2.0) | (0.9) | (0.2) | (206.3) |
| | 23182 | 2.35 | 46.0 | (17.8) | (40.8) | (14.4) | (5.7) | (3.7) | (1.5) | (0.4) | (239.4) |
| | 20104 | 3.85 | 28.1 | (39.9) | (23.5) | (11.8) | (5.2) | (2.0) | (0.9) | (0.1) | (213.5) |

TABLE IV (continued)

| Formulation | Animal No. | Weight kg. | Dose mg./kg. | mcg. of Drug* Equivalents/ml. of Serum (Fluorometric Results in Parenthesis) | | | | | | | |
|---|---|---|---|---|---|---|---|---|---|---|---|
| | | | | Time in Hours | | | | | | | 0-48 Hr. AUC** |
| | | | | 2 | 4 | 6 | 10 | 14 | 23 | 48 | |
| Polysorbate 80:-Drug* (2.5:1) Capsule | 20161 | 2.50 | 44.0 | (10.3) | (3.9) | (1.8) | (0.8) | (0.4) | (0.2) | (0.1) | (44.4) |
| | 23124 | 4.30 | 25.6 | (0.3) | (5.5) | (3.6) | (1.0) | (0.7) | (0.3) | (0.1) | (37.6) |

* $^{14}C$-Labeled p-hexadecylaminobenzoic acid sodium salt

**AUC = Area under the serum drug concentration - time curve (in mcg.-hr./ml.)

TABLE V

Summary of the Drug Concentrations Determined by the Fluorometric Assay From the Bioavailability Study in Fasted and Fed Monkeys

| Formulation | Animal No. | Weight kg. | Dose (mg./kg.) | Percent Food Consumption | | mcg./ml. Time in Hours | | | | | | | | 0-48 Hr. AUC** |
|---|---|---|---|---|---|---|---|---|---|---|---|---|---|---|
| | | | | Time of Dosing | 5 Hours Post Dose | 0 | 2 | 4 | 6 | 10 | 14 | 24 | 48 | |
| Control | 18631 | 5.70 | 37.1 | 100 | 100 | 0.0 | 1.0 | 2.5 | 2.7 | 4.1 | 1.9 | 3.0 | 0.3 | 99.4 |
| | 23130 | 3.85 | 54.9 | 20 | 85 | 0.0 | 1.1 | 5.4 | 3.3 | 1.4 | 1.3 | 0.5 | 0.1 | 47.3 |
| | 23206 | 2.70 | 73.3 | 5 | 20 | 0.0 | 2.3 | 1.5 | 1.2 | 0.6 | 0.2 | 0.1 | 0.1 | 17.9 |
| | 20098 | 5.00 | 42.5 | FAS | TED | 0.0 | 2.3 | 3.0 | 1.8 | 0.8 | 0.5 | 0.3 | 0.0 | 27.8 |
| | 23116 | 3.95 | 53.5 | FAS | TED | 0.0 | 7.2 | 5.2 | 2.9 | 1.3 | 0.6 | 0.4 | 0.0 | 49.7 |
| | 18723 | 2.70 | 73.3 | FAS | TED | 0.0 | 17.9 | 7.1 | 4.0 | 3.3 | 1.3 | 0.4 | 0.2 | 93.5 |
| Polysorbate 80:Drug* (5:1) Capsule | 18633 | 5.00 | 43.2 | 100 | 100 | 0.0 | 11.5 | 14.9 | 8.6 | 6.6 | 6.0 | 1.4 | 0.3 | 174.4 |
| | 13664 | 3.55 | 54.7 | 40 | 60 | 0.0 | 17.2 | 30.3 | 19.8 | 13.4 | 11.4 | 2.4 | 0.7 | 337.0 |
| | 20136 | 2.70 | 80.0 | 10 | 20 | 0.0 | 3.7 | 22.7 | 24.1 | 16.6 | 7.0 | 3.4 | 1.1 | 311.5 |
| | 20161 | 2.90 | 74.5 | 35 | 50 | 0.0 | 16.8 | 24.4 | 10.6 | 3.4 | 2.3 | 1.5 | 0.6 | 176.6 |
| | 18639 | 4.95 | 43.6 | FAS | TED | 0.0 | 18.6 | 55.7 | 18.7 | 7.1 | 4.4 | 2.2 | 0.3 | 304.9 |
| | 18718 | 2.30 | 77.1 | FAS | TED | 0.0 | 23.0 | 70.8 | 34.3 | 14.0 | 6.3 | 2.6 | 0.6 | 442.0 |

* $^{14}C$-Labeled p-hexadecylaminobenzoic acid sodium salt
**AUC = Area under the serum drug concentration - time curve (in mcg.-hr./ml.)

Example 1

A 5 g. portion of p-hexadecylaminobenzoic acid sodium salt is added to a solution of one ml. of polysorbate 80 in 15 ml. of water and mixed thoroughly. Evaporation of the water leaves a suspension of the drug in polysorbate 80 which is suitable for oral administration.

Example 2

A 550 mg. portion of p-hexadecylaminobenzoic acid sodium salt is placed in a glass mortar. A 17,783 mg. portion of polysorbate 80 is added slowly with constant trituration. The resulting suspension may be administered orally.

Example 3

A 550 mg. portion of p-hexadecylaminobenzoic acid sodium salt is placed in a glass mortar. A 500 mg. portion of polysorbate 80 is added and mixed with a mortar. The resulting paste is filled in gelatin capsules suitable for oral administration.

Example 4

A mixture of 550 mg. of p-hexadecylaminobenzoic acid sodium salt, 500 mg. of polysorbate 80 and 55 mg. of lecithin are mixed thoroughly in a glass mortar. The resulting paste is filled in gelatin capsules suitable for oral administration.

Example 5

An oil-in-water emulsion was prepared by mixing 35 gm. sesame oil, 3 gm. Atmul 845, and 7 gm. polysorbate 60, and slowly adding 40 gm. water with good agitation, then passing the resulting mass through a hand homogenizer. To this was added with agitation a suspension of 5 gm. p-hexadecylaminobenzoic acid sodium salt in 8 gm. water, to obtain a suspension of the active component in an oil-in-water emulsion. The resulting suspension may be administered orally.

WE CLAIM:

1. A composition of matter in pharmaceutical oral dosage form comprising a compound of the formula:

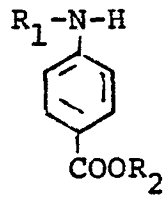

wherein $R_1$ is an unbranched or branched alkyl group $C_nH_{2n+1}$ wherein n is 8 to 19 and $R_2$ is selected from the group consisting of hydrogen, lower alkyl, benzyl, dilower alkylaminoethyl and lower alkoxyethyl together with the pharmaceutically acceptable salts thereof and non-ionic surfactant.

2. The composition of Claim 1 wherein the percentage of said compound is up to 90% dosage weight of the percentage of non-ionic surfactant is at least about 10% of the dosage weight.

3. The composition of Claim 2 wherein the percentage of said compound is about 3% to 29%.

4. The composition of Claim 1 or 2 or 3 wherein the non-ionic surfactant is polysorbate 60.

5. The composition of Claim 1 or 2 or 3 wherein the non-ionic surfactant is polysorbate 65.

6. The composition of Claim 1 or 2 or 3 wherein the non-ionic surfactant is polysorbate 80.

7. The composition of Claim 1 or 2 or 3 wherein the non-ionic surfactant is polysorbate 85.

8. A method of treating hyperlipidemia in a warm-blooded animal which comprises administering to said animal an anti-hyperlipidemia effective amount of a compound of the formula:

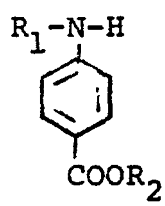

wherein $R_1$ is an unbranched or branched alkyl group $C_nH_{2n+1}$

wherein n is 8 to 19 and $R_2$ is selected from the group consisting of hydrogen, lower alkyl, benzyl, dilower alkylaminoethyl and lower alkoxyethyl together with the pharmaceutically acceptable salts thereof and non-ionic surfactant.

9. A method of treating atherosclerosis in a warm-blooded animal which comprises administering to said animal an anti-atherosclerosis effective amount of a compound of the formula:

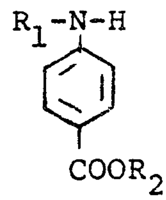

wherein $R_1$ is an unbranched or branched alkyl group $C_nH_{2n+1}$ wherein n is 8 to 19 and $R_2$ is selected from the group consisting of hydrogen, lower alkyl, benzyl, dilower alkylaminoethyl and lower alkoxyethyl together with the pharmaceutically acceptable salts thereof and non-ionic surfactants.

10. A method for increasing absorption of a compound from the alimentary tract into the circulatory system comprising introducing said compound into the alimentary tract wherein said compound is selected from those of the formula:

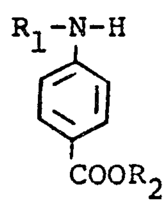

wherein $R_1$ is an unbranched or branched alkyl group $C_nH_{2n+1}$ wherein n is 8 to 19 and $R_2$ is selected from the group consisting of hydrogen, lower alkyl, benzyl, dilower alkylaminoethyl and lower alkoxyethyl together with the pharmaceutically acceptable salts thereof and is in combination with a non-ionic surfactant.

European Patent Office

# PARTIAL EUROPEAN SEARCH REPORT

which under Rule 45 of the European Patent Convention shall be considered, for the purposes of subsequent proceedings, as the European search report

Application number

0031603

EP 80201115.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.³) |
|---|---|---|---|
| | US - A - 3 932 659 (GREEN,MORGAN)<br>+ Columns 1,2 +<br>-- | 1 | A 61 K 31/245 |
| | US - A - 3 238 103 (VOGENTHALER)<br>+ Example 2 +<br>-- | 1-7 | |
| | FR - M - 638 (DRACO)<br>+ Pages 4,5 +<br>-- | 1-3 | |
| | GB - A - 889 225 (AB BOFORS)<br>+ Totality +<br>-- | 1-3 | TECHNICAL FIELDS SEARCHED (Int. Cl.³) |
| | GB - A - 1 528 386 (SCHERICO LTD.)<br>+ Examples 1-4 +<br>---- | 1-7 | A 61 K 9/00<br>A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7

Claims searched incompletely:

Claims not searched: 8-10

Reason for the limitation of the search:

Method for treatment of the human or animal body by therapy (Art.52 (4) EPC).

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| VIENNA | 09-03-1981 | MAZZUCCO |

EPO Form 1505.1 06.78